# EUROPÄISCHE PATENTANMELDUNG

(11) **EP 2 286 860 A1**
(43) Veröffentlichungstag der Anmeldung: **23.02.2011**
(21) Anmeldenummer: 10014581.2
(22) Anmeldetag: 03.01.2003
(51) Int. Cl.: A61M 15/00

(54) **Arzneimittelpulverpatrone und damit ausgestatteter Inhalator**

(30) Priorität: 24.01.2002 DE 10202940
(62) Teilanmeldung aus: 03731664.3
(71) Anmelder: MEDA Pharma GmbH & Co. KG, 61352 Bad Homburg (DE)
(72) Erfinder: Goede, Joachim, 63457 Hanau (DE); Herder, Martin, 63110 Rodgau (DE); Lange, Karl-Heinz, 32257 Bunde (DE); Eilbracht, Meike, 60314 Frankfurt (DE)
(74) Vertreter: Polypatent

(57) **Zusammenfassung**

Zur Verbesserung bei der Applikation pulverförmiger Arzneimittel wird eine Arzneimittelpatrone (1) für Pulverinhalatoren zur Aufnahme eines Arzneimitteldepots für eine Vielzahl von Arzneimitteldepots für eine Vielzahl von Arzneimittelpulverdosen vorgeschlagen mit wenigstens einem Vorratsraum (6) und einer integrierten Dosiereinrichtung, wobei die integrierte Dosiereinrichtung wenigstens einen Dosierschieber (9, 13, 14) umfasst, der zumindest aus einer Füllstellung in eine Entleerungsstellung annähernd quer zur Ausflussrichtung des Arzneimittelpulvers aus dem wenigstens einen Vorratsraum (6) annähernd transversal in einem Dosierschieberkanal (12) bewegbar ist, bei der der Dosierschieberkanal (12) mit dem wenigstens einen Dosierschieber (9, 13, 14) zumindest in der Füllstellung des Dosierschieber s (9, 13, 14) zur Umgebung abgedichtet ist, sowie weitere Maßnahmen und ein entsprechender Inhalator.

## Beschreibung

Die Erfindung betrifft eine Arzneimittelpulverpatrone für Pulverinhalatoren zur Aufnahme eines Arzneimitteldepots für eine Vielzahl von Arzneimittelpulverdosen mit wenigstens einem Vorratsraum und einer integrierten Dosiereinrichtung, wobei die integrierte Dosiereinrichtung wenigstens einen Dosierschieber umfasst, der zumindest aus einer Füllstellung in eine Entleerungsstellung annähernd quer zur Ausflussrichtung des Arzneimittelpulvers aus dem wenigstens einen Vorratsraum annähernd transversal in einem Dosierschieberkanal bewegbar ist und einen entsprechend ausgestatteten Inhalator.

### Hintergrund der Erfindung

Auf dem Gebiet der Behandlung von Bronchialerkrankungen aber auch anderen Erkrankungen, bei denen eine Medikation über den Atemwegstrakt erfolgen kann, ist es bekannt, neben der Zerstäubung von Lösungen oder Suspensionen zu inhalierbaren Aerosolen pulverförmige Medikamente zu applizieren. Beispiele für solche Medikamente sind in der Literatur vielfach beschrieben, rein exemplarisch verweisen wir auf WO 93/11773, EP 0 416 950 A1 und EP 0 416 951 A1.

Eine gebräuchliche Applikationsform ist dabei die Zuführung über ein Inhalationsgerät (Inhalator).

Bei Inhalatoren für pulverförmige Arzneimittel sind sowohl solche zur Applikation einer Einzeldosis bekannt, als auch Inhalationsgeräte, die einen Vorrat für eine Mehrzahl von Arzneimitteldosen aufweisen. Bei letzteren ist es bekannt, entweder getrennte Vorratsräume für jeweils eine einzelne Dosis vorzusehen oder einen einzelnen Aufnahmeraum zur Aufnahme einer Vielzahl von Dosen eines Medikaments.

Bei Inhalatoren, bei denen eine Vielzahl von Einzeldosen in separaten Vorratsräumen vorgesehen ist, sind solche bekannt, bei denen einzelne Räume des Inhalators jeweils mit einer Arzneimitteldosis befüllt sind. Ein Beispiel eines solchen Inhalators ist in US 5,301,666 A beschrieben. Es ist aber auch bekannt, eine Vielzahl von Arzneimittelpulverdosen jeweils getrennt in Räumen sogenannter Blisterverpackungen unterzubringen. Ein Beispiel für eine solche Blisterverpackung zur Verwendung mit einem Inhalator ist in DE 44 00 083 C2 beschrieben. Eine solche Blisterverpackung, die zugleich als Einweginhalator ausgebildet ist, ist beispielsweise in DE 44 00 084 A1 beschrieben.

Ein Inhalationsgerät, in das Blisterverpackungen eingelegt werden können, die jeweils getrennte Vorratsräume für einzelne Dosen eines pulverförmigen Arzneimittels enthalten und die mit Hilfe des Inhalationsgerätes nacheinander geleert werden können, ist beispielsweise in DE 195 23 516 C1 beschrieben.

Beispiele von Inhalatoren mit einem Vorratsraum für eine Vielzahl von Arzneimitteldosen sind im Stand der Technik vielfältig beschrieben. Ein Beispiel mit einem auswechselbaren Vorratsbehälter ist in DE-PS 846 770 beschrieben, ein weiteres in WO 95/31237.

Ein wesentliches Problem bei Inhalationssystemen, bei denen eine Vielzahl von Dosen eines medizinisch wirksamen Stoffes in einem gemeinsamen Vorratsraum untergebracht ist, ist die Zumessung einer einzelnen Dosis für einen einzelnen Inhalationsvorgang. Hierzu sind eine Vielzahl von Lösungsvorschlägen gemacht worden, wie beispielsweise in US 2,587,215 A und US 4,274,403 A beschrieben. Andere Formen von Anordnungen zum Dosieren einer einzelnen Arzneimittelpulverdosis aus einem Vorratsraum für eine Vielzahl von Arzneimitteldosen sind weiterhin in WO 92/09322, WO 93/16748 und DE 35 35 561 C2 sowie in GB 2 165 159 A beschrieben. Eine auswechselbare Patrone zur Aufnahme einer Vielzahl von Dosen eines Arzneimittelpulvers mit einem integrierten Dosierschieber ist aus DE 195 22 415 A1 bekannt.

Ein weiteres wesentliches Problem bei der Inhalation von Arzneimittelpulver ist die Zerlegung der galenischen Pulverformulierungen in lungengängige Partikel. Die auf diesem Wege verabreichten Wirkstoffe werden in aller Regel mit Trägerstoffen zusammengeführt, um eine vernünftige Dosierbarkeit des medizinisch wirksamen Stoffes zu erreichen und um weitere Eigenschaften des Arzneimittelpulvers einzustellen, die z. B. die Lagerfähigkeit betreffen können.

Lösungsansätze für Ausbildungen bei Pulverinhalatoren, mit denen die Bereitstellung lungengängiger Partikel in einem Luftstrom zur Inhalation erreicht werden soll, sind beispielsweise in EP 0 640 354 A2, US 5,505,196 A, US 5,320,714 A, US 5,435,301 A, US 5,301,666 A, DE 195 22 416 A1 und WO 97/00703 beschrieben. Dabei sind auch Vorschläge bekannt, Hilfsenergie zur Erzeugung des Luftstromes einzusetzen, beispielsweise aus ZA-A 916741.

Ganz allgemein ist es auch bei Einsatz von Medikamenten zur Inhalation in Pulverform ist es bekannt, Wirkstoffe durch Applikation vorgefertigter Wirkstoffmischungen zu kombinieren. Entsprechende Vorschläge finden sich in EP 0 416 951 A1 und WO 93/11773 beispielsweise für die Kombination von Salmeterol und Fluticasone oder Formoterol und Budesonid.

In WO 00/74754 und vielen anderen Publikationen über mehr als zwanzig Jahre ist beschrieben, dass ein erhebliches Problem hinsichtlich Feuchtigkeit insbesondere bei Pulverinhalatoren besteht. Dabei kann Feuchtigkeit sich nicht nur nachteilig auf die pharmazeutisch wirksame Medikamentenzusammensetzung wirken, sondern insbesondere das Zusammenspiel von physikalischen und chemischen Parametern der Kombination aus Wirkstoff und Hilfsstoffen beeinträchtigen. Dadurch können zum Beispiel Verklumpungen auftreten oder die Zerlegung des inhalierten Pulvers in lungengängige Partikel gestört werden. Alle diese Umstände können zu Problemen hinsichtlich der Dosierbarkeit und der Wirksamkeit der Verabreichung eines Medikamentenpulvers führen.

Zur Verringerung dieser Nachteile wurde in der Vergangenheit bereits verschiedentlich versucht, das Eindringen von Feuchtigkeit in einen Pulverinhalator durch Verwendung von Dichtungen zu verringern. Weiterhin wurde versucht, die nachteiligen Auswirkungen eingedrungener Feuchtigkeit dadurch zu vermindern, dass Trockenmittel vorgesehen wurden, um die Feuchtigkeit zu absorbieren, insbesondere die Luftfeuchtigkeit in Vorratskammern niedrig zu halten.

### Stand der Technik

Unter Bezug auf älteren Stand der Technik wird in WO 00/74754 ausführlich beschrieben, dass dort dieses Problem regelmäßig nur durch Einsatz von Trockenmitteln in verschiedener Ausgestaltung versucht worden sei zu lösen.

Die Anmelderin nimmt für sich in Anspruch, dieses Problem erstmals durch vorsehen einer Dichtung gelöst zu haben, die ein Eindringen von Feuchtigkeit in den Inhalator, insbesondere den Vorratsbehälter eines Pulverinhalators mit insbesondere elastischen Dichtungselementen verhindern soll.

Dazu wird auf Dichtelemente aus ,,allen üblicherweise bekannten Materialien, beispielsweise natürlichem oder synthetischem Gummi, einem Silicon oder PTFE" und ähnliche Materialien verwiesen.

Nachfolgend wird unter Bezugnahme auf den Pulverinhalator "Clickhaler" von Innovata Biomed eine Anordnung näher beschrieben, die sich auf eine besondere Anordnung bezüglich des Dosiermechanismus zu diesem Inhalator bezieht, der eine Dosiereinrichtung nach Art einer Zellenradschleuse in Form eines geneigten Kegelstumpfes umfasst.

Als Dichtungselement soll dabei bei der beschriebenen Ausführungsform eine ebenfalls kegelstumpfförmige Dichtungsmanschette vorgesehen sein, die über den Dosierkegelstumpf gesteckt wird und die drehbar sein soll, so dass sie eine dichtende und eine nichtdichtende Stellung einnehmen kann. Dabei wird interessanterweise auf Seite 5 beschrieben, dass diese Dichtungshülse vorzugsweise aus einem Kunststoffmaterial wie dem des Dosierkegels hergestellt sein soll. Weiterhin wird vorgeschlagen, bei der Dichtungshülse genauso viele Löcher vorzusehen, wie das Dosierkegelelement Dosierkavitäten besitzt. Dabei soll die Dichtungshülse und das Dosierelement so ausgebildet sein, dass beim Drehen beider zuerst eine durch eine Öffnung in der Dichtungshülse gebildete Dosierkammer das Medikament aus dem Vorrat aufnimmt und dann bei weiterem Verdrehen an die Dosierkavität in dem eigentlichen Dosierkegel abgibt und schließlich von dort in einen Luftkanal abgegeben wird.

Als besonders vorteilhaft wird eine Ausführungsform beschrieben, bei der die Außenkontur der Dichtungshülse einen Kugelabschnitt bildet und mit einer entsprechenden Krümmung des Medikamentenreservoirs eine gute Passung ergibt. Die Innenkontur der Dichtungshülse soll dem Kegelstumpf des Dosierkegels angepasst sein.

Aus US 6,132,394 A ist bekannt, in einer Medikamentenkammer eines inhalators einen separaten Behälter enthaltend ein Trockenmittel (Desiccant) vorzusehen. Dabei wird als Unterschied zu US 4,274,403 A beschrieben, einen vollständig geschlossenen separaten Behälter aus einem möglichst feuchtigkeitsdurchlässigen Material zu verwenden, in dem sich das Trockenmittel, zum Beispiel Silicagel befinden soll. Wesentlicher Vorteil soll dabei sein, dass gegenüber den herkömmlichen Trockenkapseln keine Montage- oder verbindungsstellen bestehen, durch die geringe Mengen des Trockenmittels in die Medikamentenkammer gelangen und dadurch das Medikamentenpulver verunreinigen können. Solche verbindungsstellen sollen bei herkömmlichen Trockenkapseln insbesondere zwischen Kapselkörper und poröser Membran bestehen, durch die der Wasserdampf in das Trockenmittel gelangen soll.

Der separate Behälter soll danach möglichst aus einem einzigen Material vorzugsweise hoher Wasserdampfdurchlässigkeit hergestellt sein. Als geeignete Werkstoffe werden Polycarbonat (PC) und ABS (Acrylnitrilbutadienstyrol) vorgeschlagen. Das Trocknungsverhalten über einen längeren Zeitraum soll dabei über das Material des Behälters angepasst werden.

Aus WO 01/46038 ist bekannt, einen Stopfen, eine Folie, eine Tablette oder eine Auskleidung aus einem EVA-Copolymer mit 35-80 Gewichts-% eines Trockenmittels wie Silicagel, Lehm oder Zinkchlorid als Trockenmittelkapsel oder eingebettet in einen Aufbewahrungsbehälter insbesondere für abgepackte Nahrungsmittel zu verwenden, wobei bei höheren Konzentrationen an Trockenmittel auf eine mangelhafte mechanische Stabilität des Stopfens etc. und die Gefahr des mechanischen Zerfalls hingewiesen wird. Dabei weisen die als geeignet beschriebenen EVA-Sorten recht hohe Anteile an Venylacetat-Copolymeren auf, so dass sich bei diesen Werkstoffen sehr hohe Wasserdampfdurchlässigkeiten einstellen.

Aus WO 01/21238 ist es bekannt, bei einem Pulverinhalator eine hermetische Abdichtung bei Nichtgebrauch vorzusehen. Dazu ist beschrieben, bei einem Pulverinhalator mit einem Medikamentenvorrat und einem unterhalb des Vorratsraumes durchgeführten Luftkanal jeweils auf beiden Seiten des Vorratsbehälters eine Dichtschürze vorzusehen, die eine Lufteintritts- und eine Inhalationsöffnung des Luftkanals in einer Ruhestellung abdeckt. Wird über eine Betätigungskappe ein Dosierstößel durch den Vorratsraum betätigt, um eine Medikamentendosis aus dem Vorrat in den Luftkanal zu befördern, werden die beiden an der Betätigungskappe befestigten Dichtschürzen mit herunterbewegt, bis der Dosierstößel seine Entleerungsstellung erreicht hat. Dabei sind Durchgangslöcher in den Dichtschürzen vorgesehen, die so angeordnet sind, dass sie in der Endstellung der Betätigungskappe die Öffnungen des Luftkanals freigeben. Solange die Betätigungskappe niedergedrückt bleibt, kann die Luft durch den Luftkanal gesogen werden. Wird die Betätigungskappe losgelassen und kehrt diese in ihre Ausgangsstellung zurück, werden die Öffnungen des Luftkanals wieder verschlossen.

über eine zusätzliche Kulissenführung und eine elastische Ausbildung der Dichtschürzen werden diese dabei gegen die Außenwandung gepresst, um die Dichtwirkung zu steigern. Bei dieser Anordnung werden die Dichtschürzen mit zunehmend zurückgelegtem Weg von der Öffnungsstellung in die Schließstellung immer stärker quer zur Bewegungsrichtung gegen die Außenwandung des Inhalators gepresst. Weiterhin ist eine elastische Dichtung in Form eines Faltenbalges zwischen Betätigungskappe und Inhalatorgehäuse vorgesehen, um den zwischen den genannten Bauteilen befindlichen Spalt zu verschließen.

### Zusammenfassung der Erfindung

Der Erfindung liegt daher die Aufgabe zugrunde, bekannte Systeme zur Applikation pulverförmiger Arzneimittel zu verbessern.

Diese Aufgabe wird erfindungsgemäß gelöst durch eine Arzneimittelpulverpatrone für Pulverinhalatoren zur Aufnahme eines Arzneimitteldepots für eine Vielzahl von Arzneimittelpulverdosen mit wenigstens einem Vorratsraum und einer integrierten Dosiereinrichtung, wobei die integrierte Dosiereinrichtung wenigstens einen Dosierschieber umfasst, der zumindest aus einer Füllstellung in eine Entleerungsstellung annähernd quer zur Ausflussrichtung des Arzneimittelpulvers aus dem wenigstens einen Vorratsraum annähernd transversal in einem Dosierschieberkanal bewegbar ist, bei der der Dosierschieberkanal mit dem wenigstens einen Dosierschieber zumindest in der Füllstellung des Dosierschiebers zur Umgebung abgedichtet ist.

Durch die erfindungsgemäße Ausbildung wird bei geringstem zusätzlichem Aufwand ein wirksamer Schutz des Arzneimittelvorrats vor Feuchtigkeit aus der Umgebung insbesondere bei der Zwischenlagerung während der Gebrauchsdauer nach Anbruch des Vorrats durch einen Patienten erhalten. Dieser Vorzug besteht sowohl, während die Arzneimittelpatrone in einem Inhalator eingesetzt ist, als auch wenn sie außerhalb des inhalators aufbewahrt wird. Eine Kompatibilität zu bekannten Pulverinhalatoren für austauschbare Arzneimittelpulverpatronen der eingangs erwähnten Art kann erhalten bleiben.

In einer besonders bevorzugten Ausführungsform ist eine erfindungsgemäße Arzneimittelpulverpatrone dadurch gekennzeichnet, dass der Dosierschieberkanal an seinem einen Ende zur Umgebung hin eine Öffnung aufweist, durch die ein Teil des Dosierschiebers hindurchtreten kann und um die Öffnung eine Anlagefläche für eine Dichtung vorgesehen ist.

Eine besonders zuverlässige Funktion kann erhalten werden, wenn der Dosierschieber eine in einer Ebene annähernd quer zu seiner Bewegungsrichtung aus der Füllstellung in die Entleerungsstellung vorgesehene Dichtfläche aufweist. Dabei kann zugleich eine Veränderung von Reibungskräften bei der Bewegung des Dosierschiebers vermieden werden, die bei bekannten Inhalatoren durch eine Bewegung der Dichtung entlang der Dichtfläche durch Pulverreste oder Verschleiß der Dichtung entstehen können.

Eine besonders zuverlässige Abdichtung wird erhalten, wenn die Abdichtung durch eine elastische Dichtung gebildet ist.

Eine besonders dauerhafte und wirksame Abdichtung bei längerer Lagerung vor Einsetzen der Arzneimittelpulverpatrone in einen Inhalator ist gewährleistet, wenn der Dosierschieber ferner in eine zusätzliche Lagerungsstellung bewegbar ist und die Dichtung zumindest in der Lagerungsstellung des Dosierschiebers dichtend elastisch vorgespannt ist, insbesondere, wenn der Dosierschieber in der Lagerungsstellung durch federelastische Mittel festgelegt ist.

In einer zweckmäßigen Ausführungsform ist eine erfindungsgemäße Arzneimittelpulverpatrone dadurch gekennzeichnet, dass die Dosiereinrichtung zumindest eine Dosierkavität zur Aufnahme einer vorbestimmten Menge eines Arzneimittelpulvers umfasst.

Für die Verabreichung von wirkstoffkombinationen kann es ferner vorteilhaft sein, wenn die Arzneimittelpulverpatrone wenigstens zwei Vorratsräume aufweist, insbesondere, wenn die Arzneimittelpulverpatrone eine Dosiereinrichtung aufweist, wobei die Dosiereinrichtung für jede der Vorratsräume eine Dosierkavität zur Zumessung einer vorbestimmten Menge eines jeden in den Vorratsräumen vorgesehenen medizinisch wirksamen Stoffes hat. Dabei ist es je nach vorgesehener Wirkstoffkombination auch vorteilhaft, wenn die Dosiereinrichtungen der einzelnen Arzneimittelpulverpatronen Dosierkavitäten mit gleichem oder unterschiedlichem Volumen aufweisen.

Ein besonders wirtschaftlicher Einsatz besonders bei teuren Arzneimittelpulvern bei nur gelegentlicher Applikation ist möglich, wenn die Arzneimittelpulverpatrone ferner eine Einrichtung zur Anzeige der in den Vorratskammern verbliebenen oder aus den Vorratskammern entnommenen Menge an Arzneimitteldosen aufweist.

Die Vorzüge der Erfindung lassen sich insbesondere bei Langzeitgebrauch nutzen bei einer Arzneimittelpulverpatrone für Pulverinhalatoren zur Aufnahme eines Arzneimitteldepots für eine Vielzahl von Arzneimittelpulverdosen mit wenigstens einem Vorratsraum und einer integrierten Dosiereinrichtung, wobei die integrierte Dosiereinrichtung zumindest eine Füllstellung und eine Entleerungsstellung annehmen kann und aus der Füllstellung in die Entleerungsstellung bewegbar ist, bei der eine Dichtung vorgesehen ist, die den Vorratsraum wenigstens in der Füllstellung der Dosiereinrichtung gegen Feuchtigkeitseintritt aus der Umgebung weitgehend abdichtet, wobei die Dichtung bei einer Bewegung der Dosiereinrichtung aus seiner Entleerungsstellung in seine Füllstellung elastisch verformbar ist ohne gleitende Relativbewegung der Dichtung gegenüber den Dichtflächen.

In einer bevorzugten Ausführungsform der Erfindung ist die Dichtung aus einem Silikonkautschuk oder einem Elastomer hergestellt ist, weiter vorzugsweise aus einem thermoplastischen Elastomer, vorzugsweise einem TPE-E (Thermoplastischen polyesterelastamer).

Die erfindungsgemäße Verbesserung der Gebrauchseigenschaften insbesondere durch wirksame Verminderung der Einwirkung von Feuchtigkeit auf ein Arzneimittelpulver während der Gebrauchsdauer durch einen Patienten oder eine Krankenanstalt wird ferner erhalten bei einer Arzneimittelpulverpatrone für Pulverinhalatoren oder einem Pulverinhalator mit wenigstens einem Vorratsraum zur Aufnahme eines Arzneimitteldepots für eine Vielzahl von Arzneimittelpulverdosen enthaltend einen Gehäusekörper und einen Deckel, die im wesentlichen den wenigstens einen Vorratsraum umschließen, bei denen der Gehäusekörper und/oder der Deckel überwiegend aus einem PVDC (Polyvenylidenchlorid), einem ganz oder teilweise mit PVDC beschichteten arzneimittelverträglichen Kunststoff, einem Olefincopolymer mit heterocyclischen Seitengruppen (COC oder mPP), oder einem PCTFE (Polytrichlorethylen) besteht.

In einer besonders vorteilhaften Ausführungsform ist eine erfindungsgemäße Arzneimittelpulverpatrone dadurch gekennzeichnet, dass wenigstens ein Dosierschieber als Bestandteil der Dosiereinrichtung überwiegend aus einem PVDC (Polyvenylidenchlorid), einem ganz oder teilweise mit PVDC beschichteten arzneimittelverträglichen Kunststoff, einem Olefincopolymer mit heterocyclischen Seitengruppen, einem zumindest teilweise orientierten PP (Polypropylen) oder einem PCTFE (Polytrichlorethylen) besteht.

Zur Begrenzung der Auswirkungen von in die Patrone eingedrungener oder darin befindlicher Feuchtigkeit auf ein Arzneimittelpulver ist ferner zweckmäßig, wenn eine Arzneimittelpulverpatrone für Pulverinhalatoren oder ein erfindungsgemäßer Pulverinhalator gekennzeichnet ist dadurch, dass der Gehäusekörper und/oder Deckel auf zumindest einem Teil der dem Vorratsraum zugewandten Seite ein Blend aus mit in einer thermoplastischen Matrix eingebettetem Trockenmittel umfasst.

Für eine Vermeidung von Beeinträchtigungen des Arzneimittels durch Trockenmittelreste ist es erfindungsgemäß vorteilhaft, eine Arzneimittelpulverpatrone für Pulverinhalatoren oder einen Pulverinhalator mit wenigstens einem Vorratsraum zur Aufnahme eines Arzneimitteldepots für eine Vielzahl von Arzneimittelpulverdosen enthaltend zumindest einen Formkörper aus einem Blend einer thermoplastischen Matrix mit darin eingebettetem Trockenmittel, vorzugsweise Silicagel, Bentonite oder Molekularsieb, vorzusehen, insbesondere, wenn in einer Matrix aus einem Thermoplasten geringer Wasseraufnahme Kanäle gebildet sind, wie sie durch Auslösen löslicher Koextrudatkomponenten erhältlich sind. Für eine schnelle Aufnahme von Restfeuchtigkeit in dem Vorratsraum kann es dabei auch zweckmäßig sein, wenn in einer Matrix aus einem Thermoplasten geringer Wasseraufnahme Wasserdampf aufnehmende Fasern als Füllstoff eingebettet sind.

Wirtschaftlich für eine massenweise Produktion ist es besonders vorteilhaft, wenn der Blend in einer Matrix aus einem Thermoplasten geringer Wasseraufnahme und einem darin eingebetteten Trockenmittel zumindest als Teil einer Innenwandung eines Vorratsraumes durch Mehrkomponentenspritzguss in einem Gehäusekörper aus einem im wesentlichen wasserdampfundurchlässigen Kunststoff ausgebildet ist.

Im Sinne der Erfindung ist es weiterhin vorteilhaft, wenn Gehäusekörper und Deckel wasserdicht versiegelt sind, vorzugsweise durch Ultraschallschweißen.

Besonders wirtschaftlich in der Herstellung ist es, wenn die Dichtung an den Gehäusekörper oder den Dosierschieber mit angespritzt ist.

Wirtschaftlich lässt sich die Erfindung vorteilhaft umsetzen bei einem Inhalator für pulverförmige Arzneimittel mit einer erfindungsgemäßen Arzneimittelpulverpatrone, sowie mit einem Inhalator für pulverförmige Arzneimittel, bei dem das Arzneimittel mittels eines Luftstromes von einem Patienten aufgenommen werden kann, der gekennzeichnet ist durch eine Aufnahme für eine erfindungsgemäße Arzneimittelpulverpatrone.

Die Vorzüge der Erfindung kommen insbesondere für die behandlungsbedürftigen Patienten besonders zum Tragen bei einer erfindungsgemäßen Arzneimittelpulverpatrone enthaltend ein Pulver mit einem oder mehreren der folgenden Wirkstoffe: Analgetika, Antiallergika, Antibiotika, Anticholinergika, Antihistaminika, antiinflammatorisch wirkende Substanzen, Antipyretika, Kortikoide, Steroide, Antitussiva, Bronchodilatatoren, Diuretika, Enzyme, Herz-Kreislauf wirksame Substanzen, Hormone, Proteine und Peptide.

### Anwendung der Erfindung

Mit der Erfindung ist es möglich, pharmakodynamisch aktive Wirkstoffe in Form von pulverigen Arzneimitteln auch bei erhöhter Empfindlichkeit gegen Feuchtigkeit oder unter klimatisch ungünstigen Umgebungsbedingungen über einen langen Gebrauchszeitraum bereitzustellen und dabei auch die Vorzüge des Einsatzes mehrfach verwendbarer Inhalatoren mit auswechselbaren Arzneimittelpulverpatronen zu erhalten. Weiterhin ist es auch möglich, pulverförmige Arzneimittel in verschiedenen Wirkstoffkombinationen unter verbesserten Aufbewahrungsbedingungen zur Inhalation bereitzustellen, von denen einzelne Wirkstoffe eine erhöhte Feuchtigkeitsempfindlichkeit hinsichtlich der Lagerfähigkeit, ihrer Beständigkeit oder ihrer Dosierbarkeit aufweisen. Wirkstoffe, für die die Erfindung einsetzbar ist, können ferner beispielsweise sein aus der Gruppe der Betasympatikomimetika: Salbutamol, Reproterol, Fenoterol, Formoterol, Salmeterol. Beispiele aus der Gruppe der Kortikosteroide können sein: Budesonid, Beclomethason, Fluticason, Triamcinolon, Loteprednol, Mometason, Flunisolid, Ciclosonid. Beispiele aus der Gruppe der Anticholinergica können sein: Ipatropiumbromid, Tiotropiumbromid, Glycopyrrolate.

Beispiele aus der Gruppe der Analgetica und Migränetherapeutika können sein: Morphin, Tramadol, Flupirtin, Sumatryptan. Aus der Gruppe der Peptide und Proteine können beispielsweise verwendet werden: Cetrorelix, Insulin, Calcitonin, Parathyroid Hormon, Faktor VIII Analoge, Alpha Interferon, Beta Interferon, Heparin, FSH (Follikel Stimulierendes Hormon), Collistin, Tobramycin.

Die Anwendung ist nicht auf die hier genannten Wirkstoffe beschränkt. Das beschriebene Arzneimittelpulverpatrone eignet sich für alle Wirkstoffe, die in Pulverform dosiert und inhalativ appliziert werden können. Durch entsprechende Modifikation des Systems und der Dosiervorrichtung eignet sich die beschriebene Erfindung auch zur Kombination von Wirkstoffen, die flüssige Zubereitungen, zum Beispiel Lösungen oder Suspensionen von pharmakodynamisch aktiven Wirkstoffen, enthalten.

Arzneimittelpulverformulierungen, die sinnvoll mit dem Arzneimittelpulverpatronensystem gemäß der Erfindung verwendet werden können, können verschiedene Wirkstoffe enthalten, wie beispielsweise Analgetika, Antiallergika, Antibiotika, Anticholinergika, Antihistaminika, antiinflammatorisch wirkende Substanzen, Antipyretika, Kortikoide, Steroide, Antitussiva, Bronchodilatatoren, Diuretika, Enzyme, Herz-Kreislauf wirksame Substanzen, Hormone, Proteine und Peptide. Beispiele für Analgetika sind Codein, Diamorphin, Dihydromorphin, Ergotamin, Fentanyl und Morphin; Beispiele für Antiallergika sind Cromoglicinsäure und Nedocromil; Beispiele für Antibiotika sind Cephalosporine, Fusafungin, Neomycin, Penicilline, Pentamidin, Streptomycin, Sulfonamide und Tetracycline, Collistin, Tobramycin; Beispiele für Anticholinergika sind Atropin, Atropinmethonitrat, Ipratropiumbromid, Oxitropiumbromid, Trospiumchlorid und Tiotropiumbromid; Beispiele für Antihistaminika sind Azelastin, Flezelastin und Methapyrilen; Beispiele für antiinflammatorisch wirksame Substanzen sind Beclometason, Budesonid, Loteprednol, Dexamethason, Flunisolid, Fluticason, Tipredane, Triamcinolon, Mometason; Beispiele für Antitussiva sind Narcotin und Noscapin; Beispiele für Bronchodilatatoren sind Bambuterol, Bitolterol, Carbuterol, Clenbuterol, Ephedrin, Epinephrin, Formoterol, Fenoterol, Hexoprenalin, Ibuterol, Isoprenalin, Isoproterenol, Metaproterenol, Orciprenalin, Phenylephrin, Phenylpropanolamin, Pirbuterol, Procaterol, Reproterol, Rimiterol, Salbutamol, Salmeterol, Sulfonterol, Terbutalin und Tolobuterol; Beispiele für Diuretika sind Amilorid und Furosemid; ein Beispiel für Enzyme ist Trypsin; Beispiele für Herz-Kreislauf wirksame Substanzen sind Diltiazem und Nitroglycerin; Beispiele für Hormone sind Cortison, Hydrocortison und Prednisolon; Beispiele für Proteine und Peptide sind Cyclosporine, Cetrorelix, Glucagon und Insulin. Weitere Wirkstoffe, die eingesetzt werden können, sind Adrenochrom, Colchicin, Heparin, Scopolamin. Die beispielhaft angeführten Wirkstoffe können als freie Basen oder Säuren oder als pharmazeutisch verträgliche Salze eingesetzt werden. Als Gegenionen können beispielsweise physiologisch verträgliche Erdalkali- oder Alkalimetalle oder Amine sowie beispielsweise Acetat, Benzolsulfonat, Benzoat, Hydrogencarbonat, Hydrogenartrat, Bromid, Chlorid, Iodid, Carbonat, Citrat, Fumarat, Malat, Maleat, Cluconat, Lactat, Pamoat und Sulphat eingesetzt werden. Es können auch Ester eingesetzt werden, beispielsweise Acetat, Acetonid, Propionat, Dipropionat, valerat.

Die Erfindung ermöglicht eine auch über einen längeren Zeitraum sehr genau auf den Patienten abgestimmte Dosierung durch den Arzt vorzunehmen, ohne dass eine hinsichtlich der Behandlungskosten nachteilige Entsorgung teilentleerter Patronen erforderlich wäre oder eine Kompatibilität mit anderen Patronen mit unterschiedlichen Dosiereinrichtungen behindert wäre, z.B. mit der aus WO 97/00703 bekannten Patrone.

Kurzbeschreibung der Zeichnungen

Es zeigen:
- Figur 1: eine erfindungsgemäße Arzneimittelpulverpatrone in perspektivischer Ansicht;
- Figur 2: eine Draufsicht auf eine Dichtung einer erfindungsgemäßen Arzneimittelpulverpatrone;
- Figur 3: eine Ansicht eines Mitnehmers eines Dosierschiebers einer erfindungsgemäßen Arzneimittelpulverpatrone;
- Figur 4A: eine Ansicht eines Dosierschieberkörpers einer erfindungsgemäßen Arzneimittelpulverpatrone;
- Figur 4B: einen Längsschnitt durch den Dosierschieberkörper einer erfindungsgemäßen Arzneimittelpulverpatrone aus Figur 4A;
- Figur 5: einen Längsschnitt durch eine erfindungsgemäße Arzneimittelpulverpatrone in einem Inhalator mit dem Dosierschieber in der Entleerungsstellung; und
- Figur 6: einen Längsschnitt durch eine erfindungsgemäße Arzneimittelpulverpatrone in einem Inhalator mit dem Dosierschieber in der Befüllungsstellung.

Beschreibung von bevorzugten Ausführungsbeispielen

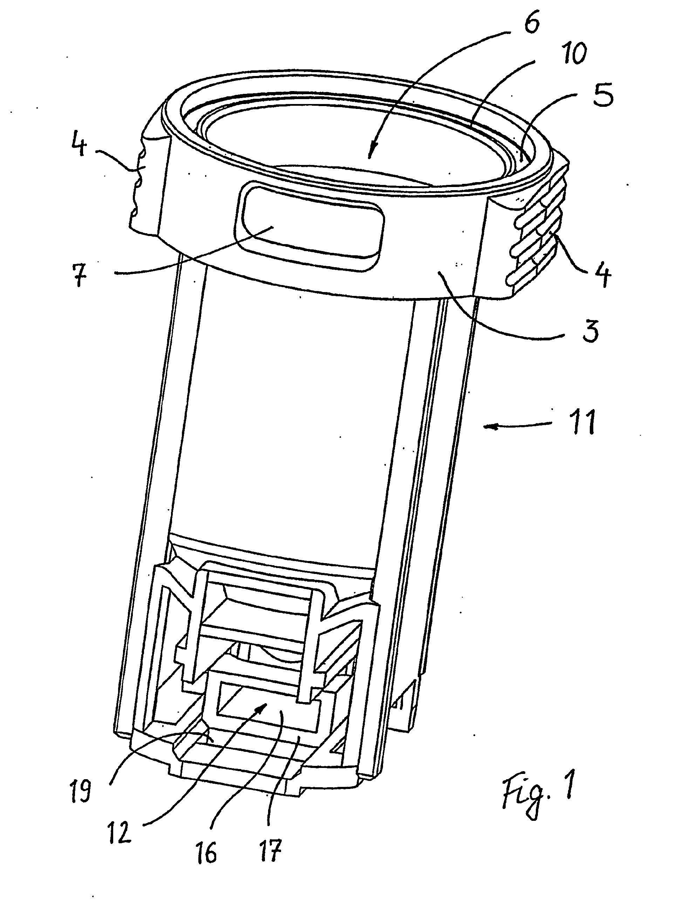

Fig. 1 zeigt eine perspektivische Ansicht eines Gehäusekörpers 11 einer erfindungsgemäßen

Arzneimittelpulverpatrone 1 zum austauschbaren Einsetzen in einen Pulverinhalator 2. Die dargestellte Arzneimittelpulverpatrone 1 weist an dem oberen Bereich ihres Gehäusekörpers 11 einen Rand 3 auf, der zwei Griffbereiche 4 umfasst, um ein bequemes Einsetzen der Arzneiznittelpulverpatrone 1 in einen Pulverinhalator 2 zu ermöglichen. Bei dem dargestellten Ausführungsbeispiel ist zugleich in dem Rand 3 in darin gebildeten Ringkanal 5 eine Einrichtung zur Anzeige der in dem Vorratsraum 6 verbliebenen oder aus dem Vorratsraum 6 entnommenen Menge an Arzneimitteldosen vorgesehen (jedoch nicht im einzelnen dargestellt), z.B. als Folienstreifen mit entsprechenden Markierungen ausgebildet, wie im einzelnen in WO 97/00703 beschrieben. Die Markierungen sind dann durch das Sichtfenster 7 in dem Rand 3 vom Benutzer abzulesen.

Der Rand 3 dient auch zur Aufnahme eines Deckels 8 mit dem der den wesentlichen Teil der Arzneimittelpulverpatrone 1 bildenden Vorratsraum 6 verschließbar ist. Zweckmäßig wird ein solcher Deckel 8 mit einem innerhalb des Randes 3 gebildeten umlaufenden Kragen 10 wasserdicht versiegelt, beispielsweise durch Ultraschallschweißen.

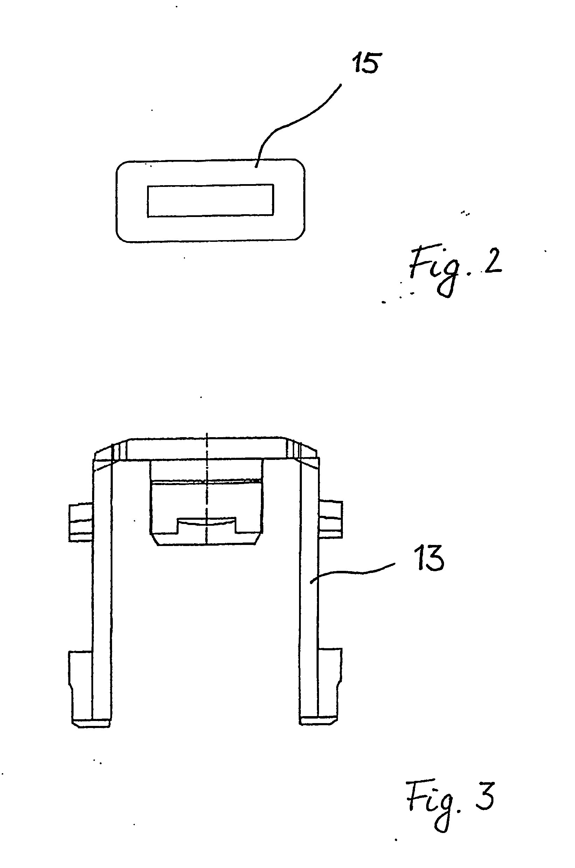

Unterhalb des Vorratsraumes 6 ist ein Dosierschieberkanal 12 angeordnet, in dem als Dosiereinrichtung ein Dosierschieber beweglich angeordnet ist, der bei dem hier beschriebene Ausführungsbeispiel dreiteilig aus einem Mitnehmer 13, dem eigentlichen Dosierschieberkörper 14 und einer Dichtung 15 gebildet ist (Figuren 2, 3 und 4A und 4B). Der Dosierschieber wird dadurch gebildet, dass die in Figur 2 gezeigte Dichtung 15 in den in Figur 3 dargestellten Mitnehmer 13 eingesetzt der Mitnehmer 13 mit Dichtung 15 auf den Dosierschieberkörper 14 aufgeklipst wird.

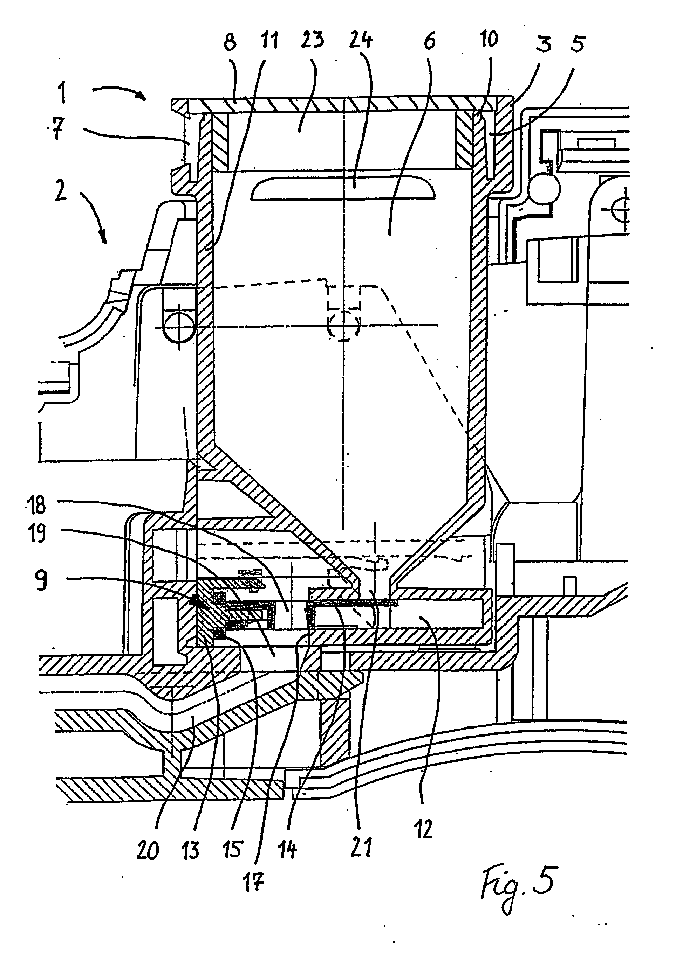

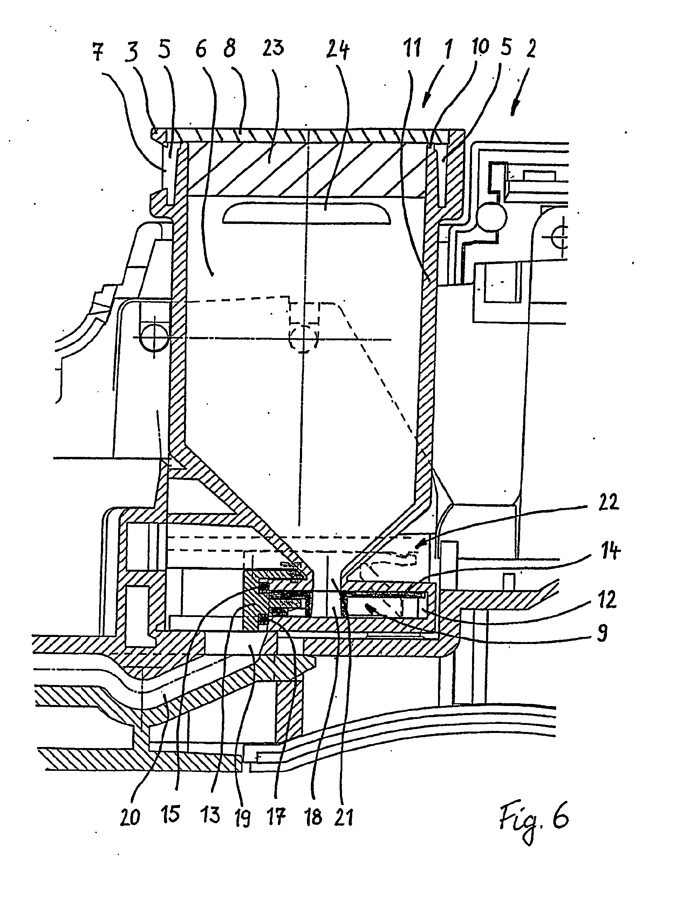

Wie aus Figur 1 gut zu entnehmen ist, hat der Dosierschieberkanal 12 an seinem einen Ende eine Öffnung 16 und um die Öffnung 16 herum ist eine Anlagefläche 17 für die Dichtung 15 des Dosierschiebers gebildet. Die Anlagefläche 17 ist zugleich als Dichtfläche vorgesehen und erstreckt sich in einer Ebene annähernd senkrecht zu der Bewegungsrichtung des Dosierschiebers aus einer Füllstellung, wies sie in Figur 5 dargestellt ist, in eine Entleerungsstellung, wie sie in Figur 6 zu sehen ist.

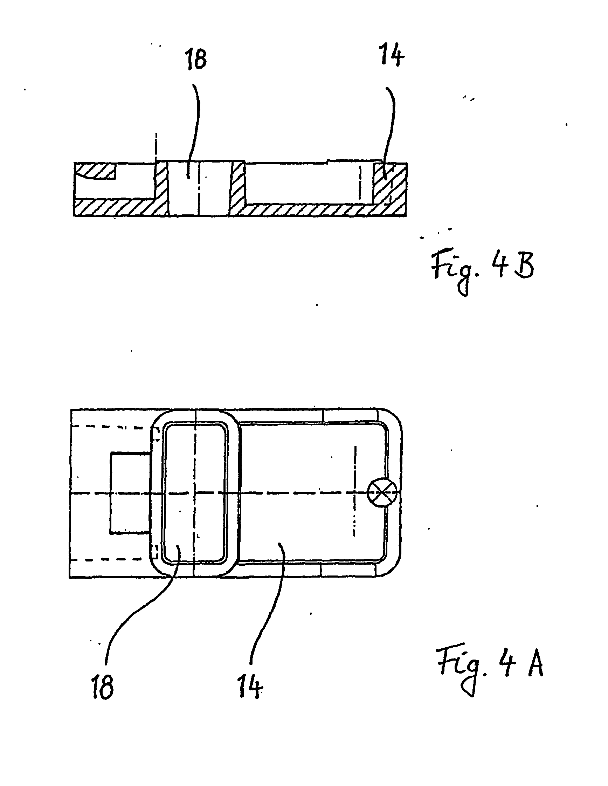

Der in den Figuren 4A und 4B dargestellte Dosierschieberkörper 14 umfasst eine Dosierkavität 18, deren Aufnahmevolumen die für eine Inhalation bereitzustellende Dosiermenge bildet. Die Dichtung 15 kann beispielsweise auch im Mehrkomponenten-Spritzguss mit angespritzt sein und dazu z.B. aus einem thermoplastischen Elastomer bestehen. Entsprechend kann eine Dichtfläche aus an dem Dosierschieber vorgesehen sein und die elastische Dichtung 15 im Bereich der Öffnung 16 des Dosierschieberkanals 12 montiert oder noch besser angespritzt sein.

Der Gehäusekörper 11 und/oder der Deckel 8 und/oder der Dosierschieberkörper 14 können vorteilhaft aus einem COC durch Spritzgießen hergestellt sein. Ein geeignetes Material ist unter der Bezeichnung TOPAS® 8007 als Versuchsprodukt von der Firma Ticona in Deutschland erhältlich.

Für Arzneimittelkombinationen, bei denen die Pulver nicht oder schlecht als Gemisch aufbewahrt werden können, kann es auch zweckmäßig sein, zwei Vorratskammern anstelle des einen Vorratsraumes 6 vorzusehen.

In den Figuren 5 und 6 ist ein Längsschnitt durch die Arzneimittelpulverpatrone 1 dargestellt wie sie in einen Inhalator 2 eingesetzt ist. Wie in den Figuren zu erkennen ist, ist der Dosierschieber, insgesamt mit 9 bezeichnet, in dem Dosierschieberkanal 12 zumindest aus der in Figur 6 dargestellten Füllstellung die in Figur 5 dargestellte Entleerungsstellung verschiebbar.

In der in Figur 6 dargestellten Befüllungsstellung kann Arzneimittelpulver aus dem Vorratsraum 6 in die Dosierkavität 18 fallen. Wenn die Dosierkavität 18 mit einem Arzneimittelpulver wie gewünscht gefüllt sind, kann der Dosierschieber 9 durch nur schematisch angedeutete Eingriffsmittel eines Pulverinhalators, wie z.B. in US 5,840,279 A beschrieben, die mit dem Mitnehmer 13 zusammenwirken, in die in Figur 5 dargestellte Entleerungsposition verschoben werden.

Die Entleerungsstellung ist dann erreicht, wenn die Dosierkavität 18 sich über einer Entleerungsöffnung 19 befindet. Wenn der Dosierschieber 9 diese Stellung erreicht hat, kann das Arzneimittelpulver aus der Dosierkavitäten 18 durch die Entleerungsöffnung 19 z.B. in einen Pulverkanal 20 eines Inhalators 2 fallen.

In Figur 6 ist gut die Befüllungsstellung des Dosierschiebers 9 erkennbar mit der Dosierkavität 18 unter einem Loch 21 der Unterseite des Vorratsraumes 6. Zum Erreichen der Entleerungsstellung wird der Dosierschieber 9 in Figur 6 so weit nach links verschoben, bis sich diese Dosierkavität 18 mit der Entleerungsöffnung 19 deckt und das Arzneimittelpulver nach unten herausfallen kann.

Weiterhin ist in Figur 6 gut zu erkennen, dass die Dichtung 15 des Dosierschiebers 9 an der Anlagefläche 17 des Dosierschieberkanals 12 anliegt und bei vorzugsweise leichter elastischer Verformung für eine gute Abdichtung sorgt. Dies kann durch Vorspannung mittels federelastischer Mittel erfolgen, insbesondere über eine Betätigungseinrichtung des Inhalators für den Dosierschieber 9, die zweckmäßig zudem eine unmittelbare Rückkehr des Dosierschiebers 9 aus der Entleerungsstellung, wie in Figur 5 dargestellt, in seine gedichtete Befüllungsstellung, wie sie in Figur 6 gezeigt ist, bewirkt, sobald eine Arzneimitteldosis entnommen wurde.

Für einen höheren Anpressdruck der Dichtung 15 des Dosierschiebers 9 an der Anlagefläche 17 des Dosierschieberkanals 12 und damit für eine besonders zuverlässige Abdichtung bei der Lagerung einer erfindungsgemäßen Arzneimittelpulverpatrone insbesondere vor dem erstmaligen Einsetzen in einen Pulverinhalator ist es vorteilhaft, bezogen auf die Darstellung in Figur 6 noch etwas weiter rechts eine zusätzliche Lagerungsstellung für den Dosierschieber 9 einer gefüllten Arzneimittelpulverpatrone 1 vorzusehen, in der der Dosierschieber 9 durch die angedeutete lösbare Schnappverbindung 22 festlegbar ist. In dieser Lagerungsstellung ist dazu die Dichtung 15 des Dosierschiebers 9 an der Anlagefläche 17 des Dosierschieberkanals 12 einer erhöhten Vorspannkraft ausgesetzt.

Im oberen Bereich des Vorratsraumes ist vorteilhaft noch ein Formkörper 23 eingesetzt, der vorzugsweise über entsprechende Formkanten 24 in seiner Lage gesichert ist, um eine mechanische Belastung des Arzneimittelpulvers zu vermeiden. Der Formkörper 23 ist zweckmäßig durch Spritzgießen aus einem Blend aus einer thermoplastischen Matrix und einem Trockenmittel hergestellt. Das Trockenmittel soll dabei insbesondere in dem Vorratsraum 6 befindliche oder über die Dosierkavität 18 eindringende Feuchtigkeit aufnehmen. Durch die Verwendung eines solchen Formkörpers 23 ist sichergestellt, dass keine Krümel des Trockenmittels, typischerweise Silicagel, in das Arzneimittel und damit in die Atemwege eines Patienten gelangen kann. Ein solcher Formkörper kann aus einer PP-Matrix bestehen, die selbst kein Wasser aufnimmt und die mit einem wasserlöslichen Compound und dem Trockenmittel gemischt gespritzt wird, z.B. Polyethylenglykol, und die wasserlösliche Komponente anschließend ausgewaschen wird. Dadurch entsteht eine schwammartige Struktur mit Kanälen, die nach dem Trocknen des Formkörpers eine schnelle Wasseraufnahme des (nicht wasserlöslichen) Silicagels durch große Oberflächen unter Ausnutzung auch einer Kapillarkondensation ermöglichen.

Um eine schnelle Wasseraufnahme in dem gesamten Formkörper 23 zu erhalten, kann es auch zweckmäßig sein, geeignete Fasern als Füllstoff in dem Blend aus Trockenmittel und thermoplastischer Matrix einzubetten, die durch ihre Kapillarwirkung einen raschen Transport der Luftfeuchtigkeit bzw. des Wasser zu de Trockenmittel bewirken.

Der Formkörper 23 kann auch in Form einer Wandauskleidung nach Art eines Einsatzes, wie rein beispielsweise in Figur 5 angedeutet ausgebildet werden oder durch Merkkomponentenspritzguss bei der Herstellung der Arzneimittelpulverpatrone ganz oder teilweise eine Innenwandung des Vorratsraumes 6 bilden.

### Aspekte

1. Arzneimittelpulverpatrone (1) für Pulverinhalatoren zur Aufnahme eines Arzneimitteldepots für eine Vielzahl von Arzneimittelpulverdosen mit wenigstens einem Vorratsraum (6) und einer integrierten Dosiereinrichtung, wobei die integrierte Dosiereinrichtung wenigstens einen Dosierschieber (9, 13, 14) umfasst, der zumindest aus einer Füllstellung in eine Entleerungsstellung annähernd quer zur Ausflussrichtung des Arzneimittelpulvers aus dem wenigstens einen Vorratsraum (6) annähernd transversal in einem Dosierschieberkanal (12) bewegbar ist,
   dadurch gekennzeichnet, dass
   der Dosierschieberkanal (12) mit dem wenigstens einen Dosierschieber (9,13, 14) zumindest in der Füllstellung des Dosierschiebers (9, 13, 14) zur Umgebung abgedichtet ist.
2. Arzneimittelpulverpatrone (1) nach Aspekt 1,
   dadurch gekennzeichnet, dass
   der Dosierschieberkanal (12) an seinem einen Ende zur Umgebung hin eine Öffnung (16) aufweist, durch die ein Teil des Dosierschiebers (9, 13,14) hindurchtreten kann und um die Öffnung (16) eine Anlagefläche (17) für eine Dichtung (15) vorgesehen ist.
3. Arzneimittelpulverpatrone (1) nach Aspekt 2,
   dadurch gekennzeichnet, dass
   der Dosierschieber (9, 13,14) eine in einer Ebene annähernd quer zu seiner Bewegungsrichtung aus der Füllstellung in die Entleerungsstellung vorgesehene Dichtfläche aufweist.
4. Arzneimittelpulverpatrone (1) nach einem der vorhergehenden Aspekte,
   dadurch gekennzeichnet, dass
   die Abdichtung durch eine elastische Dichtung (15) gebildet ist.
5. Arzneimittelpulverpatrone (1) nach einem der vorhergehenden Aspekte,
   dadurch gekennzeichnet, dass
   der Dosierschieber (9, 13,14) ferner in eine Lagerungsstellung bewegbar ist und die Dichtung (15) zumindest in der Lagerungsstellung des Dosierschiebers (9,13, 14) dichtend elastisch vorgespannt ist.
6. Arzneimittelpulverpatrone (1) nach Aspekt 5,
   dadurch gekennzeichnet, dass
   der Dosierschieber (9,13, 14) in der Lagerungsstellung durch federelastische Mittel festgelegt ist.
7. Arzneimittelpulverpatrone nach einem der vorhergehenden Aspekte,
   dadurch gekennzeichnet, dass
   die Dosiereinrichtung zumindest eine Dosierkavität (18) zur Aufnahme einer vorbestimmten Menge eines Arzneimittelpulvers umfasst.
8. Arzneimittelpulverpatrone nach einem der vorhergehenden Aspekte,
   dadurch gekennzeichnet, dass
   das Arzneimittelpulverpatrone wenigstens zwei Vorratsräume (6) aufweist.
9. Arzneimittelpulverpatrone nach Aspekt 8,
   dadurch gekennzeichnet, dass
   die Arzneimittelpulverpatrone eine Dosiereinrichtung aufweist, wobei die Dosiereinrichtung für jede der Vorratsräume (6) eine Dosierkavität (18) zur Zumessung einer vorbestimmten Menge eines jeden in den Vorratsräumen (6) vorgesehenen medizinisch wirksamen Stoffes hat.
10. Arzneimittelpulverpatrone nach einem der vorhergehenden Aspekte,
   dadurch gekennzeichnet, dass
   die Dosiereinrichtungen der einzelnen Arzneimittelpulverpatronen Dosierkavitäten (18) mit gleichem oder unterschiedlichem Volumen aufweisen.
11. Arzneimittelpulverpatrone nach einem der vorhergehenden Aspekte,
   dadurch gekennzeichnet, dass
   die Arzneimittelpulverpatrone (1) ferner eine Einrichtung zur Anzeige der in den Vorratsräumen (6) verbliebenen oder aus den Vorratsräumen (6) entnommenen Menge an Arzneimitteldosen aufweist.
12. Arzneimittelpulverpatrone (1) für Pulverinhalatoren zur Aufnahme eines Arzneimitteldepots für eine Vielzahl von Arzneimittelpulverdosen mit wenigstens einem Vorratsraum (6) und einer integrierten Dosiereinrichtung, wobei die integrierte Dosiereinrichtung zumindest eine Füllstellung und eine Entleerungsstellung annehmen kann und aus der Füllstellung in die Entleerungsstellung bewegbar ist,
   dadurch gekennzeichnet, dass
   eine Dichtung (15) vorgesehen ist, die den Vorratsraum (6) wenigstens in der Füllstellung der Dosiereinrichtung gegen Feuchtigkeitseintritt aus der Umgebung weitgehend abdichtet, wobei die Dichtung (15) bei einer Bewegung der Dosiereinrichtung aus seiner Entleerungsstellung in seine Füllstellung elastisch verformbar ist ohne gleitende Relativbewegung der Dichtung (15) gegenüber den Dichtflächen (17).
13. Arzneimittelpulverpatrone nach einem der vorhergehenden Aspekte,
   dadurch gekennzeichnet, dass
   die Dichtung (15) aus einem Silikonkautschuk oder einem Elastomer hergestellt ist
14. Arzneimittelpulverpatrone nach einem der vorhergehenden Aspekte,
   dadurch gekennzeichnet, dass
   die Dichtung (15) aus einem thermoplastischen Elastomer, vorzugsweise einem TPE-E (Thermoplastischen Polyesterelastomer), hergestellt ist.
15. Arzneimittelpulverpatrone (1) für Pulverinhalatoren oder Pulverinhalator mit wenigstens einem Vorratsraum (6) zur Aufnahme eines Arzneimitteldepots für eine Vielzahl von Arzneimittelpulverdosen. enthaltend einen Gehäusekörper (11) und einen Deckel (8), die im wesentlichen den wenigstens einen Vorratsraum (6) umschließen,
   dadurch gekennzeichnet, dass der Gehäusekörper (11) und/oder der Deckel (8) überwiegend aus einem PVDC (Polyvenylidenchlorid), einem ganz oder teilweise mit PVDC beschichteten arzneimittelverträglichen Kunststoff, einem Olefincopolymer mit heterocyclischen Seitengruppen [COC oder mPP], oder einem PCTFE (Polytrichlorethylen) besteht.
16. Arzneimittelpulverpatrone (1) nach einem der vorhergehenden Aspekte, dadurch gekennzeichnet, dass
   wenigstens ein Dosierschieber (9,13, 14) als Bestandteil der Dosiereinrichtung überwiegend aus einem PVDC (Polyvenylidenchlorid), einem ganz oder teilweise mit PVDC beschichteten arzneimittelverträglichen Kunststoff, einem Olefincopolymer mit heterocyclischen Seitengruppen, einem zumindest teilweise orientierten PP (Polypropylen) oder einem PCTFE (Polytrichlorethylen) besteht.
17. Arzneimittelpulverpatrone (1) für Pulverinhalatoren oder Pulverinhalator nach einem der vorhergehenden Aspekte,
   dadurch gekennzeichnet, dass
   der Gehäusekörper (11) und/oder Deckel (8) auf zumindest einem Teil der dem Vorratsraum (6) zugewandten Seite ein Blend aus mit in einer thermoplastischen Matrix eingebettetem Trockenmittel umfasst.
18. Arzneimittelpulverpatrone (1) für Pulverinhalatoren oder Pulverinhalator mit wenigstens einem Vorratsraum zur Aufnahme eines Arzneimitteldepots für eine Vielzahl von Arzneimittelpulverdosen enthaltend zumindest einen Formkörper (23) aus einem Blend einer thermoplastischen Matrix mit darin eingebettetem Trockenmittel, vorzugsweise Silicagel.
19. Arzneimittelpulverpatrone (1) für Pulverinhalatoren oder Pulverinhalator nach einem der Aspekte 17 oder 18,
   dadurch gekennzeichnet, dass
   in einer Matrix aus einem Thermoplasten geringer Wasseraufnahme Kanäle gebildet sind, wie sie durch Auslösen löslicher Koextrudatkomponenten erhältlich sind.
20. Arzneimittelpulverpatrone (1) für Pulverinhalatoren oder Pulverinhalator nach einem der Aspekte 17 oder 18,
   dadurch gekennzeichnet, dass
   in einer Matrix aus einem Thermoplasten geringer Wasseraufnahme Wasserdampf aufnehmende Fasern als Füllstoff eingebettet sind.
21. Arzneimittelpulverpatrone (1) für Pulverinhalatoren oder Pulverinhalator nach einem der Aspekte 15 bis 18,
   dadurch gekennzeichnet, dass
   der Blend in einer Matrix aus einem Thermoplasten geringer Wasseraufnahme und einem darin eingebetteten Trockenmittel zumindest als Teil einer Innenwandung eines Vorratsraumes (6) durch Mehrkomponentenspritzguss in einem Gehäusekörper (11) aus einem im wesentlichen wasserdampfundurchlässigen Kunststoff ausgebildet ist.
22. Arzneimittelpulverpatrone (1) nach einem der vorhergehenden Aspekte, dadurch gekennzeichnet, dass
   Gehäusekörper (11) und Deckel (8) wasserdicht versiegelt sind, vorzugsweise durch Ultraschallschweißen.
23. Arzneimittelpulverpatrone (1) nach einem der vorhergehenden Aspekte, dadurch gekennzeichnet, dass
   die Dichtung (15) an den Gehäusekörper (11) oder den Dosierschieber (9, 13, 14) mit angespritzt ist.
24. Inhalator für pulverförmige Arzneimittel mit einer Arzneimittelpulverpatrone nach einem der vorhergehenden Aspekte.
25. Inhalator für pulverförmige Arzneimittel, bei dem das Arzneimittel mittels eines Luftstromes von einem Patienten aufgenommen werden kann, gekennzeichnet durch eine Aufnahme für eine Arzneimittelpulverpatrone nach einem der Aspekte 1 bis 23.
26. Arzneimittelpulverpatrone nach einem der Aspekte 1 bis 23 enthaltend ein Pulver mit einem oder mehreren der folgenden Wirkstoffe : Analgetika, Antiallergika, Antibiotika, Anticholinergika, Antihistaminika, antiinflammatorisch wirkende Substanzen, Antipyretika, Kortikoide, Steroide, Antitussiva, Bronchodilatatoren, Diuretika, Enzyme, Herz-Kreislauf wirksame Substanzen, Hormone, Proteine und Peptide.

Diuretika, Enzyme, Herz-Kreislauf wirksame Substanzen, Hormone, Proteine und Peptide.

## Patentansprüche

1. Arzneimittelpulverpatrone (1) für Pulverinhalatoren oder Pulverinhalator mit wenigstens einem Vorratsraum (6) zur Aufnahme eines Arzneimitteldepots für eine Vielzahl von Arzneimittelpulverdosen. enthaltend einen Gehäusekörper (11) und einen Deckel (8), die im wesentlichen den wenigstens einen Vorratsraum (6) umschließen,
**dadurch gekennzeichnet, dass**
der Gehäusekörper (11) und/oder der Deckel (8) überwiegend aus einem PVDC (Polyvenylidenchlorid), einem ganz oder teilweise mit PVDC beschichteten arzneimittelverträglichen Kunststoff, einem Olefincopolymer mit heterocyclischen Seitengruppen [COC oder mPP], oder einem PCTFE (Polytrichlorethylen) besteht.

2. Arzneimittelpulverpatrone (1) nach Anspruch 1,
**dadurch gekennzeichnet, dass**
wenigstens ein Dosierschieber (9,13, 14) als Bestandteil der Dosiereinrichtung überwiegend aus einem PVDC (Polyvenylidenchlorid), einem ganz oder teilweise mit PVDC beschichteten arzneimittelverträglichen Kunststoff, einem Olefincopolymer mit heterocyclischen Seitengruppen, einem zumindest teilweise orientierten PP (Polypropylen) oder einem PCTFE (Polytrichlorethylen) besteht.

3. Arzneimittelpulverpatrone (1) für Pulverinhalatoren oder Pulverinhalator nach einem der vorhergehenden Ansprüche,
**dadurch gekennzeichnet, dass**
der Gehäusekörper (11) und/oder Deckel (8) auf zumindest einem Teil der dem Vorratsraum (6) zugewandten Seite ein Blend aus mit in einer thermoplastischen Matrix eingebettetem Trockenmittel umfasst.

4. Arzneimittelpulverpatrone (1) für Pulverinhalatoren oder Pulverinhalator mit wenigstens einem Vorratsraum zur Aufnahme eines Arzneimitteldepots für eine Vielzahl von Arzneimittelpulverdosen enthaltend zumindest einen Formkörper (23) aus einem Blend einer thermoplastischen Matrix mit darin eingebettetem Trockenmittel, vorzugsweise Silicagel.

5. Arzneimittelpulverpatrone (1) für Pulverinhalatoren oder Pulverinhalator nach einem der Ansprüche 3 oder 4,
**dadurch gekennzeichnet, dass**
in einer Matrix aus einem Thermoplasten geringer Wasseraufnahme Kanäle gebildet sind, wie sie durch Auslösen löslicher Koextrudatkomponenten erhältlich sind.

6. Arzneimittelpulverpatrone (1) für Pulverinhalatoren oder Pulverinhalator nach einem der Ansprüche 3 oder 4,
**dadurch gekennzeichnet, dass**
in einer Matrix aus einem Thermoplasten geringer Wasseraufnahme Wasserdampf aufnehmende Fasern als Füllstoff eingebettet sind.

7. Arzneimittelpulverpatrone (1) für Pulverinhalatoren oder Pulverinhalator nach einem der Ansprüche 1 bis 4,
**dadurch gekennzeichnet, dass**
der Blend in einer Matrix aus einem Thermoplasten geringer Wasseraufnahme und einem darin eingebetteten Trockenmittel zumindest als Teil einer Innenwandung eines Vorratsraumes (6) durch Mehrkomponentenspritzguss in einem Gehäusekörper (11) aus einem im wesentlichen wasserdampfundurchlässigen Kunststoff ausgebildet ist.

8. Arzneimittelpulverpatrone (1) nach einem der vorhergehenden Ansprüche,
**dadurch gekennzeichnet, dass**
Gehäusekörper (11) und Deckel (8) wasserdicht versiegelt sind, vorzugsweise durch Ultraschallschweißen.

9. Arzneimittelpulverpatrone (1) nach einem der vorhergehenden Ansprüche,
**dadurch gekennzeichnet, dass**
die Dichtung (15) an den Gehäusekörper (11) oder den Dosierschieber (9, 13, 14) mit angespritzt ist.

10. Inhalator für pulverförmige Arzneimittel mit einer Arzneimittelpulverpatrone nach einem der vorhergehenden Ansprüche

11. Inhalator für pulverförmige Arzneimittel, bei dem das Arzneimittel mittels eines Luftstromes von einem Patienten aufgenommen werden kann, **gekennzeichnet durch**
eine Aufnahme für eine Arzneimittelpulverpatrone nach einem der Ansprüche 1 bis 9.

12. Arzneimittelpulverpatrone nach einem der Ansprüche 1 bis 9 enthaltend ein Pulver mit einem oder mehreren der folgenden Wirkstoffe : Analgetika, Antiallergika, Antibiotika, Anticholinergika, Antihistaminika, antiinflammatorisch wirkende Substanzen, Antipyretika, Kortikoide, Steroide, Antitussiva, Bronchodilatatoren, Diuretika, Enzyme, Herz-Kreislauf wirksame Substanzen, Hormone, Proteine und Peptide.
